# EUROPEAN PATENT SPECIFICATION

(11) **EP 2 868 255 B1**
(45) Date of publication and mention of the grant of the patent: **03.10.2018**
(21) Application number: 13810306.4
(22) Date of filing: 07.06.2013
(51) Int. Cl.: A61B 1/04, H04N 5/367, H04N 7/18, G02B 23/24, A61B 1/00, G11B 20/24, H04N 5/243, H04N 17/00, A61B 1/045, H04N 5/225

(54) **IMAGING DEVICE AND IMAGING SYSTEM**
ABBILDUNGSVORRICHTUNG UND ABBILDUNGSSYSTEM
DISPOSITIF D'IMAGERIE ET SYSTÈME D'IMAGERIE

(30) Priority: 27.06.2012 JP 2012144564
(43) Date of publication of application: 06.05.2015
(73) Proprietor: Olympus Corporation, Tokyo 192-8507 (JP)
(72) Inventor: OKAWA, Fumiyuki, Hachioji-shi, Tokyo 192-8507 (JP); TANAKA, Yasuhiro, Hachioji-shi, Tokyo 192-8507 (JP); MATSUI, Yasunori, Hachioji-shi, Tokyo 192-8507 (JP)
(74) Representative: Gunzelmann, Rainer
(86) International application number: PCT/JP2013/065818
(87) International publication number: WO 2014/002732

(56) References cited:
- EP-A1- 1 381 227
- EP-A2- 1 143 709
- WO-A1-2012/081617
- JP-A- 2002 085 342
- JP-A- 2003 234 966
- JP-A- 2003 234 966
- JP-A- 2010 157 849
- JP-A- 2011 200 410
- US-A- 5 654 537
- US-A1- 2008 317 454
- US-A1- 2010 085 448
- US-A1- 2011 237 885

## Description

### Field

The present disclosure relates to an imaging apparatus and an imaging system capable of outputting, as image information, an electrical signal that has been photoelectrically converted from a pixel optionally designated as a target to be read from among a plurality of pixels to be imaged, for example.

### Background

In the medical field, an endoscope system is used for observing an organ of a subject such as a patient in the related art. The endoscope system includes: an inserting portion which is flexible, has a long thin shape, and configured to be inserted into a body cavity of the subject; an image pickup device (imaging apparatus) provided at a distal end of the inserting portion and configured to capture an in-vivo image; and a display unit capable of displaying the in-vivo image captured by the image pickup device. At the time of acquiring the in-vivo image by using the endoscope system, the inserting portion is inserted into the body cavity of the subject, and then an illuminating light such as a white light is emitted to a body tissue inside the body cavity from the distal end of the inserting portion, and the image pickup device captures the in-vivo image. A user such as a doctor observes the organ of the subject based on the in-vivo image displayed by the display unit.

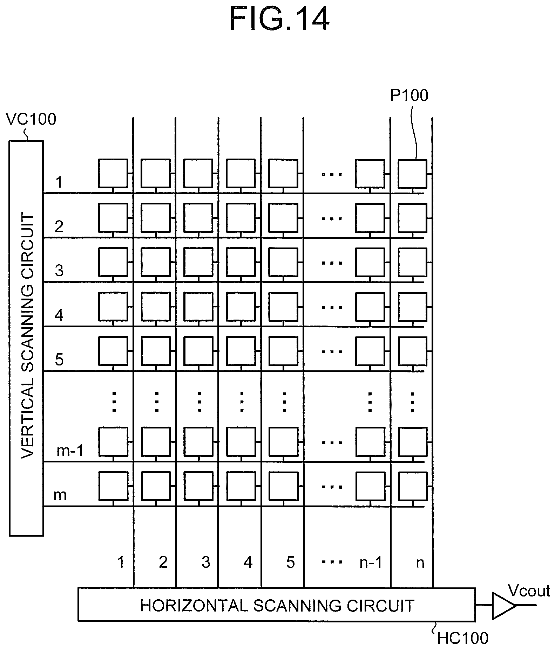

FIG. 14 is a circuit diagram illustrating a configuration of the image pickup device according to the related art. Now, in the following, a description is given for the case where the image pickup device includes a CMOS (Complementary Metal Oxide Semiconductor) image sensor. An image pickup device includes: a light receiving unit on which a plurality of pixels P100 is arranged in a two-dimensional matrix form and each of the plurality of pixels P100 includes a photodiode that photoelectrically converts light from an optical system to output an electrical signal as image information and accumulates electric charge corresponding to the light quantity and an amplifier that amplifies the electric charge accumulated by the photodiode; and a reading unit (a vertical scanning circuit VC100 (row selection circuit) and a horizontal scanning circuit HC100 (column selection circuit)) configured to read, as the image information, the electrical signal generated by the pixel optionally set as a reading target from among the plurality of pixels of the light receiving unit. The vertical scanning circuit VC100 and the horizontal scanning circuit HC100 are connected to each of the pixels P100 in order to select a pixel to be read.

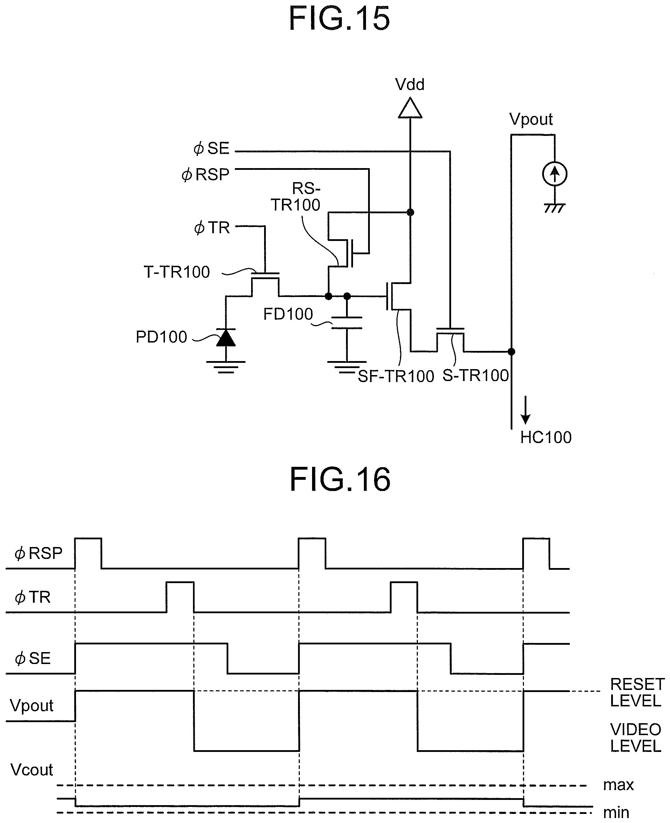

FIG. 15 is a circuit diagram illustrating a configuration of a unit pixel of the light receiving unit according to the related art. FIG. 16 is a timing chart schematically illustrating signal transmission at the image pickup device according to the related art. As illustrated in FIGS. 15 and 16, the unit pixel according to the related art includes: a photodiode PD100 that accumulates the incident light after photoelectrically converting the incident light to the signal electric charge corresponding to the light quantity; a capacitor FD100 that converts the signal electric charge transferred from the photodiode PD100 to a voltage level; a transfer transistor T-TR100 that transfers, to the capacitor FD100, the signal electric charge accumulated in the photodiode PD100 during an ON period; a reset transistor RS-TR100 that releases the signal electric charge accumulated in the capacitor FD100; a row selection transistor S-TR100 controlled to be turned ON in the case where a horizontal line including the unit pixel is selected as a line (row) to be read; and an output transistor SF-TR100 that outputs, by a source follower, a voltage level change caused by the signal electric charge transferred to the capacitor FD100 while the row selection transistor S-TR100 is in the ON state, to a specified signal line. Note that each pixel P100 is connected to a power source Vdd.

In the pixel P100 having the above-described configuration, when a reset pulse φRSP becomes high level (rises), the reset transistor RS-TR100 is controlled to be turned ON and the capacitor FD100 is reset. After that, the signal electric charge corresponding to the incident light quantity is sequentially accumulated in the photodiode PD100. Here, when the transfer transistor T-TR100 is controlled to be turned ON (when the electric charge transfer pulse φTR rises) in the pixel P100 to be read out from the light receiving unit, transfer of the signal electric charge from the photodiode PD100 to the capacitor FD100 is started. Also, the row selection transistor S-TR100 is controlled to be turned ON by the row selection pulse φSE from the vertical scanning circuit VC100 (row selection circuit), thereby outputting pixel information (signal electric charge of the photodiode PD100) of each line to the reading unit as a pixel signal in the order of reading. Further, in accordance with this pixel signal output, a pixel output voltage Vpout changes from a reset level to a video level.

Thus, signal processing such as noise reduction by use of, for example, Correlated Double Sampling is applied to the image signal from each pixel P100, and then the image signal is output to the outside as an output voltage Vcout. At this point, a signal processing unit executing the signal processing outputs a video signal at a voltage level between a maximum (max) and a minimum (min) (see FIG. 16).

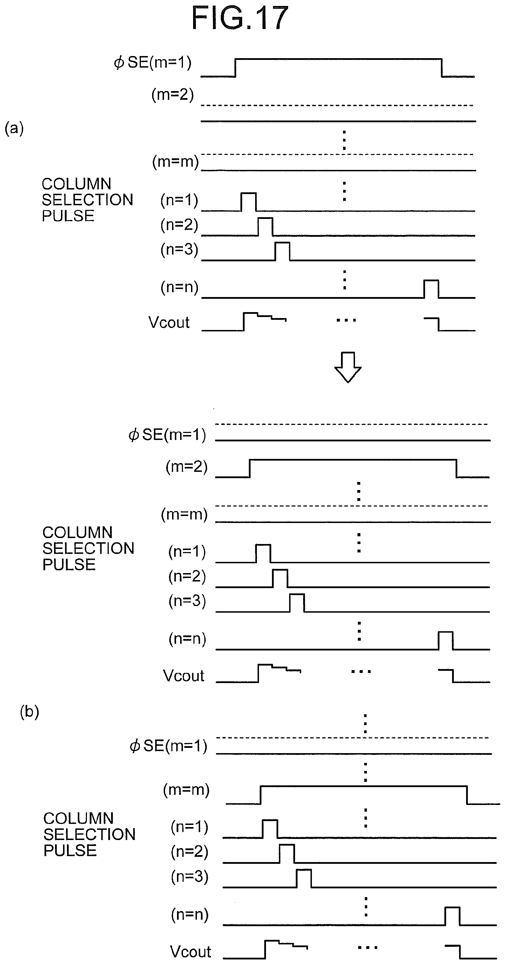

FIG. 17 is a timing chart schematically illustrating signal transmission in each row in the light receiving unit according to the related art. In the light receiving unit, a row (m) is selected by a row selection pulse φSE from the vertical scanning circuit VC100 (row selection circuit), and the pixels in the selected row sequentially output electrical signals in accordance with a column (n) number. For instance, as illustrated in (a) of FIG. 17, m = 1 is selected as the row, and a pixel signal is output from a pixel in each column in the numerical order of the column (n). After that, as illustrated in (b) of FIG. 17, the pixel signals are output from the pixels of the respective columns for the selected rows (m = 2, ···, m) .

In the case where malfunction occurs in the endoscope system having the above-described image pickup device, it is necessary to identify a failure location. Here, in the case where abnormality is occurring in a displayed image, at which component (inserting portion, imaging apparatus, and display unit) the failure is occurring can be determined by replacing each component with other so as to identify the failure location from among the above-described inserting portion, imaging apparatus, and display unit.

In Addition, for example, a technique is disclosed in which a test pattern signal for detecting abnormality of a signal or the like is generated from an imaging apparatus as a tool to identify the abnormality occurrence on the imaging apparatus side, and an image based on this test pattern signal is displayed by a display unit, thereby identifying the failure location (See Patent Literature 1, for example). Further, for example, a technology is disclosed in which presence of a missing bit in digital signal data is determined at an imaging apparatus and it is determined whether abnormality in the imaging apparatus is caused by malfunction of a CCD, or malfunction of an AFE (analog front end) that performs analog-digital conversion, etc. on the data (See Patent Literature 2, for example). Moreover, for example, a technology is disclosed in which presence of abnormality is determined based on a test pattern signal and in the case where there is abnormality occurring, correction processing for data to be transmitted is executed (See Patent Literature 3, for example).

### Citation List

### Patent Literature

Patent Literature 1: Japanese Patent Application Laid-open No. 2011-206185
Patent Literature 2: Japanese Patent Application Laid-open No. 2009-226169
Patent Literature 3: Japanese Patent Application Laid-open No. 2011-55543

EP 1 143 709 A2 concerns a method and an apparatus for testing an image sensor array such as a C-MOS imager which has sensing circuits arranged in rows and columns and wherein the sensing circuits include photosensitive devices. A reset voltage is applied to the photosensitive device in each of the sensor circuits such that at least adjacent circuits are reset to different voltage levels. The voltage on each photosensitive device is detected and compared to an expected level to determine if and where any faults may exist in the sensing circuits or lines in the array. A different reset voltage may be applied to each of the sensor circuits. One voltage level is applied to every second column to provide a supply voltage to the photosensitive devices and to every second row to generate a reset enable signal for the photosensitive devices. The second voltage level is applied to the remaining columns and rows resulting in different reset voltage levels on adjacent sensing circuits.

US20110085448A1 discloses a solid state imaging device includes a plurality of first pixels, a plurality of second pixels, a signal line, and a fixing portion. The plurality of first pixels includes a photoelectric conversion portion which converts incident light into a signal charge and accumulates the converted signal charge. The plurality of second pixels includes the photoelectric
conversion portion and is shaded so that the incident light is not incident on the photoelectric conversion portion.

EP1381227A1 discloses a solid-state image pickup device including: a pixel unit having a plurality of unit pixels that perform photoelectric conversion; a driving circuit for driving the pixel unit to control output of a pixel output signal; an output signal processing circuit for subjecting the pixel output signal outputted from the pixel unit to signal processing, and outputting a resulting pixel output signal; a pixel defect determining circuit for capturing the pixel output signal outputted from the pixel unit, and determining a pixel defect by comparing the pixel output signal with a reference signal; and a timing generator for supplying an operating pulse to the driving circuit, the output signal processing circuit, and the pixel defect determining circuit.

US2008317454 (A1) discloses an image capturing apparatus includes an image sensor, composition unit, coupling unit, and arithmetic unit. The image sensor has a plurality of pupil-divided focus detection portions each formed from a first focus detection pixel and second focus detection pixel. The composition unit composites signals output from the first and second focus detection pixels to obtain respective first and second composite signals.

WO2012081617A1 discloses an endoscopic imaging device capable of stably acquiring captured images. The device comprises: a CMOS imaging element; a timing generator and an analog front end (AFE) unit. A control circuit controls pixel information output processing from the light-receiving unit and pixel information read-out processing from the timing generator and the AFE unit.
An anomaly determination unit determines whether or not there are anomalies in the pixel information read out by
the timing generator and the AFE unit and outputs to the control circuit a notification signal when an anomaly has occurred.

### Summary

### Technical Problem

The techniques disclosed in Patent Literature 1 to Patent Literature 3 are able to determine whether abnormality occurs due to malfunction of an image sensor such as a missing bit in data and a test pattern signal or due to malfunction of AFE, but are not able to identify, in detail, an abnormality location inside the imaging apparatus having a plurality of elements.

The present invention has been made in view of the foregoing, and an object of the invention is to provide an imaging apparatus and an imaging system that are able to identify an abnormality location inside the imaging apparatus in detail.

### Solution to Problem

In order to solve the problem described above and achieve the object, an imaging apparatus is provided according to claim 1.

According to the imaging apparatus, the signal processing unit may include: a noise reduction unit configured to reduce a noise component contained in the electrical signal; an adjustment unit configured to adjust a gain of
the electrical signal to keep a constant output level; and an A/D converter configured to perform analog-digital conversion on the electrical signal output via the adjustment unit. The control unit selects one or a plurality of units from among the noise reduction unit, the adjustment unit, the A/D converter and the transmission unit, and causes each of the selected units to output the electrical signal corresponding to the specified display pattern.

According to the imaging apparatus, the light receiving unit may include an optical black area provided at a periphery of an effective pixel, and a part of the optical black area includes a pixel capable of receiving the light.

An imaging system may include: the imaging apparatus according to the above-described invention; and a processing device electrically connected to the imaging apparatus and configured to generate image data based on the processed signal transmitted from the transmission unit.

The above-described imaging system may include a relay processing unit electrically connected to each of the imaging apparatus and the processing device and configured to relay the electrical signal. The processing device causes the relay processing unit to output the electrical signal corresponding to the specified display pattern.

### Advantageous Effects of Invention

According to the invention, because a control unit controls an output mode of an electrical signal output by each pixel, on a pixel-by-pixel basis, and outputs the electrical signal corresponding to a specified display pattern (test pattern signal), it is possible to identify an abnormality location inside the imaging apparatus in detail.

### Brief Description of Drawings

FIG. 1 is a diagram illustrating a schematic configuration of an endoscope system that is an imaging apparatus.
FIG. 2 is a block diagram illustrating a functional configuration of a main part of the endoscope system.
FIG. 3 is a circuit diagram illustrating a configuration of an imaging unit of the endoscope system.
FIG. 4 is a circuit diagram schematically illustrating a configuration of an imaging unit of the endoscope system.
FIG. 5 is a circuit diagram illustrating a configuration of a unit pixel of a light receiving unit of the endoscope system.
FIG. 6-1 is a diagram illustrating an image when a specified test pattern is output from a sensor unit by pixel-by-pixel control.
FIG. 6-2 is an enlarged diagram of an area illustrated in FIG. 6-1.
FIG. 6-3 is a timing chart illustrating an output mode when the test pattern corresponding to the image illustrated in FIG. 6-1 is output.
FIG. 6-4 is a timing chart illustrating an output mode when a captured image is output according to the related art.
FIG. 7 is a schematic diagram illustrating an exemplary image corresponding to the test pattern signal in the endoscope system.
FIG. 8 is a schematic diagram illustrating an exemplary image corresponding to the test pattern signal in the endoscope system.
FIG. 9-1 is an explanatory diagram illustrating an exemplary use mode of the test pattern signal.
FIG. 9-2 is an explanatory diagram illustrating an exemplary use mode of the test pattern signal.
FIG. 9-3 is an explanatory diagram illustrating an exemplary use mode of the test pattern signal.
FIG. 9-4 is an explanatory diagram illustrating an exemplary use mode of the test pattern signal.
FIG. 9-5 is an explanatory diagram illustrating an exemplary use mode of the test pattern signal.
FIG. 10-1 is an explanatory diagram illustrating an exemplary use mode of signal transmission.
FIG. 10-2 is an explanatory diagram illustrating an exemplary use mode of signal transmission.
FIG. 11 is a schematic diagram illustrating a light receiving unit according to a modified example 1.
FIG. 12 is a schematic diagram illustrating a light receiving unit according to a modified example 2.
FIG. 13 is a block diagram illustrating a functional configuration in a main part of an endoscope system according to a modified example 3.
FIG. 14 is a circuit diagram illustrating a configuration of an image pickup device according to the related art.
FIG. 15 is a circuit diagram illustrating a configuration of a unit pixel of a light receiving unit according to the related art.
FIG. 16 is a timing chart schematically illustrating signal transmission at the unit pixel of the image pickup device according to the related art.
FIG. 17 is a timing chart schematically illustrating signal transmission in each row in the light receiving unit according to the related art.

### Description of Embodiments

A medical endoscope system that captures and displays an image inside a body cavity of a subject such as a patient will be described below as an example of an imaging system. Also, note that the same components are denoted by the same reference signs in the drawings. Furthermore, note that the drawings are schematic and the relation of the thicknesses and the widths of the respective members, the ratio of the respective members, etc. differ from the actual relation. Portions that have different sizes and ratios one another may be included among the drawings.

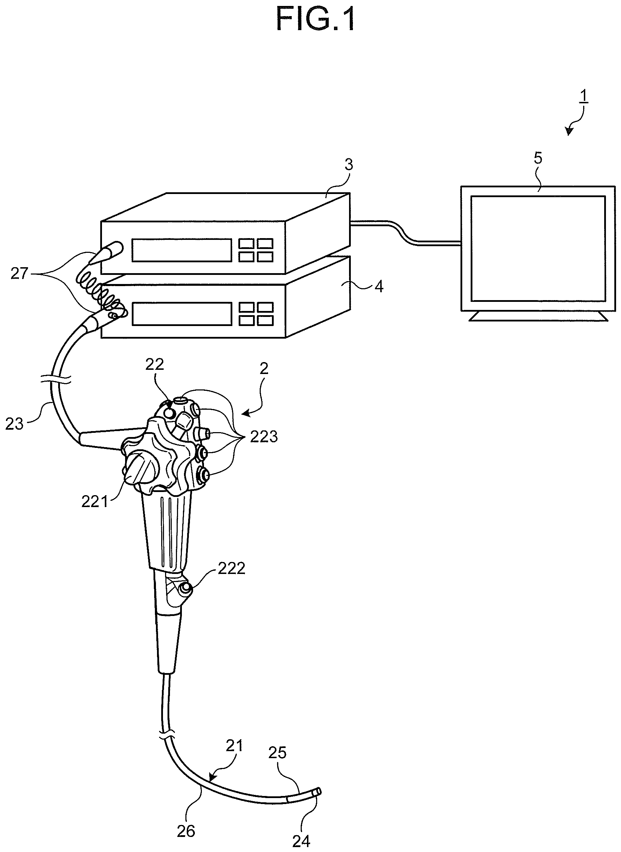

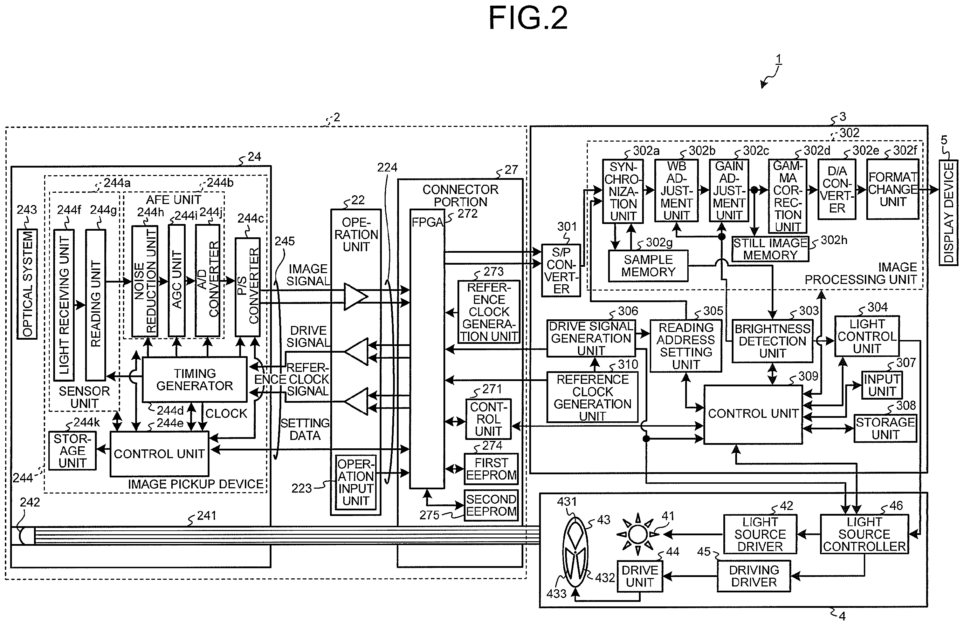

FIG. 1 is a diagram illustrating a schematic configuration of an endoscope system 1. FIG. 2 is a block diagram illustrating a functional configuration of a main part of the endoscope system 1. As illustrated in FIG. 1, the endoscope system 1 includes an endoscope 2 configured to capture an in-vivo image of a subject by inserting a distal-end portion into a body cavity of the subject, a control device 3 (processing device) configured to apply a prescribed image processing to the in-vivo image captured by the endoscope 2 and also integrally control operation of an entire portions of the endoscope system 1, a light source device 4 configured to generate illuminating light emitted from the distal end of the endoscope 2, and a display device 5 configured to display the in-vivo image applied with the image processing by the control device 3.

The endoscope 2 is connected to an inserting portion 21 having flexibility and a thin long shape and also to a proximal-end side of the inserting portion 21, and includes an operating unit 22 that receives various kinds of operation signals, and a universal cord 23 that extends in a direction different from a direction in which the inserting portion 21 extends from the operating unit 22 and includes various kinds of cables that connect the control device 3 to the light source device 4.

The inserting portion 21 includes a distal-end portion 24 including an image pickup device later described inside thereof, a freely-bendable bending portion 25 including a plurality of bending pieces, and a long-shaped flexible tube 26 connected to a proximal-end side of the bending portion 25.

The distal-end portion 24 includes: a light guide 241 formed of glass fiber and the like and constituting a guide optical path for the light generated from the light source device 4; an illumination lens 242 provided at a distal end of the light guide 241; an optical system 243 for condensing the light, an image pickup device 244 as an imaging apparatus provided at an image forming position of the optical system 243 and configured to receive the light condensed by the optical system 243, photoelectrically convert the light to an electrical signal, and apply a prescribed signal processing to the electrical signal; a cable assembly 245; and an instrument channel (not shown) where the instrument of the endoscope 2 passes through. The optical system 243 includes one or a plurality of lenses.

The configuration of the image pickup device 244 will be described with reference to FIG. 2. As illustrated in FIG. 2, the image pickup device 244 includes a sensor unit 244a (imaging unit) that photoelectrically converts the light from the optical system 243 and outputs the electrical signal as image information, an analog front end 244b (hereinafter referred to as "AFE unit 244b") configured to perform noise elimination and analog-digital conversion on the electrical signal output from the sensor unit 244a and provided as a signal processing unit and, a P/S converter 244c (transmission unit) that performs parallel-serial conversion on a digital signal (processing signal) output from the AFE unit 244b and outputs the converted signal to the outside, a timing generator 244d that generates pulses for drive timing for the sensor unit 244a and various kinds of signal processing at the AFE unit 244b and the P/S converter 244c, a control unit 244e that controls operation of the image pickup device 244, and a storage unit 244k that stores various kinds of setting information. The image pickup device 244 is a CMOS image sensor. The timing generator 244d receives various kinds of drive signals transmitted from the control device 3. Also, the control unit 244e receives, from the control device 3, signals to perform setting of reading mode (e.g., pixel addition, cutting, thinning, etc.) and setting for outputting a test pattern. It is also possible to separately provide a receiving unit that receives the various kinds of drive signals transmitted from the control device 3.

The sensor unit 244a includes a light receiving unit 244f on which photodiode that accumulates electric charge corresponding to a light quantity and a plurality of pixels that outputs the electric charge accumulated by the photodiode are arranged in a two-dimensional matrix form, and a reading unit 244g that reads, as the image information, an electrical signal generated by a pixel optionally set as a reading target from among the plurality of pixels of the light receiving unit 244f.

The AFE unit 244b includes a noise reduction unit 244h that reduces noise components contained in the electrical signal, an AGC (Auto Gain Control) unit 244i that adjusts a gain of the electrical signal to keep a constant output level as an adjustment unit, and an A/D converter 244j that performs analog-digital conversion on the electrical signal output via the AGC unit 244i. The noise reduction unit 244h reduces noise by using, for example, correlated double sampling.

The control unit 244e controls various kinds of operations of the distal-end portion 24 in accordance with setting data received from the control device 3. The control unit 244e is formed by using a CPU (Central Processing Unit) or the like. Further, the control unit 244e controls the output mode of the electrical signals output by the respective pixels of the light receiving unit 244f per pixel unit based on address information related to a reading target pixel set by a reading address setting unit 305 described later, and controls the reading unit 244g to output an electrical signal corresponding to a prescribed display pattern (test pattern).

The storage unit 244k is implemented by using a semiconductor memory such as a flash memory or a DRAM (Dynamic Random Access Memory), and stores identification information of the control device 3, observation information indicating that an observation method is a simultaneous method or a frame sequential method, an imaging speed (frame rate) of the image pickup device 244, setting information such as a pixel information reading speed of the sensor unit 244a from an optional pixel and a shutter control setting, transmission control information of the pixel information read by the AFE unit 244b, pattern information of a test pattern signal (electrical signal corresponding to a prescribed display pattern) so as to identify an abnormality location, and so on. Note that the test pattern signal includes an electrical signal corresponding to a pseudo video signal.

The cable assembly 245 in which a plurality of signal lines for transmitting and receiving the electrical signal to and from the control device 3 is bundled is connected between the operating unit 22 and the distal-end portion 24, and the cable assembly 224 is connected between the operating unit 22 and a connector portion 27. The plurality of signal lines includes a signal line to transmit an image signal output from the image pickup device 244 to the control device 3, a signal line to transmit a control signal output from the control device 3 to the image pickup device 244, and so on. Further, for transmitting/receiving the electrical signal, a transmission method (differential transmission) whereby two signal lines (differential signal lines) are used to transmit one signal is adopted. Since noise can be cancelled by setting voltages of the differential signal lines to positive (+) and negative (-, phase inversion) even when the noise is mixed, resistivity against noise is higher compared to a single end signal and therefore highspeed data transmission can be achieved, suppressing radiation noise. The above-described differential transmission is preferably used in the case where the length of the universal cord 23 or the flexible tube 26 is long. In the case where the mentioned length is short, single end signal transmission utilizing the single end signal can be adopted.

The operating unit 22 includes a bending knob 221 that bends the bending portion 25 in the vertical direction and in the horizontal direction, a treatment instrument inserting portion 222 from which a treatment instrument such as a living body forceps, a laser knife, an inspection probe or the like is inserted into the body cavity, and a plurality of switches 223 that functions as an operation input unit that inputs operation instruction signals of peripheral devices such as an air feed means, a water feed means, a gas feed means besides the control device 3 and the light source device 4. The instrument to be inserted from the instrument inserting portion 222 passes through the instrument channel of the distal-end portion 24 and is exposed from an aperture (not shown).

The universal cord 23 includes at least the light guide 241 and the cable assembly 224.

Further, the endoscope 2 is disposed at an end of a side different from a side connected to the operating unit 22 of the universal cord 23, and includes the connector portion 27 detachably attached to each of the control device 3 and the light source device 4. At the connector portion 27, the connecting part detachably connected to each of the control device 3 and the light source device 4 is electrically connected via a coil-like coil cable. The connector portion 27 includes, inside thereof, a control unit 271 that controls the endoscope 2, an FPGA (Field Programmable Gate Array) 272, a reference clock generation unit 273 that generates a reference clock signal (e.g., 68 MHz clock) to be a basis of operation in each of the components inside the endoscope 2, a first EEPROM 274 that records configuration data of the FPGA 272, and a second EEPROM 275 that stores individual data of the endoscope including imaging information. The connector portion 27 is electrically connected to each of the distal-end portion 24 (image pickup device 244) and the control device 3, and functions as a relay processing unit to relay the electrical signal. Further, as long as electrical connection is possible, connection between the connecting parts detachably connected to each of the control device 3 and the light source device 4 at the connector portion 27 is not limited to the coil cable.

Next, a configuration of the control device 3 will be described. The control device 3 includes an S/P converter 301, an image processing unit 302, a brightness detection unit 303, a light control unit 304, the reading address setting unit 305, a drive signal generation unit 306, an input unit 307, a storage unit 308, a control unit 309, and a reference clock generation unit 310. According to the present embodiment, a configuration adopting the frame sequence will be described as the control device 3, but the simultaneous method is also adoptable.

The S/P converter 301 performs serial-parallel conversion on an image signal (electrical signal) received from the distal-end portion 24 via the operating unit 22 and the connector portion 27.

The image processing unit 302 generates an in-vivo image displayed by the display device 5 based on the image signal in the parallel form output from the S/P converter 301. The image processing unit 302 includes a synchronization unit 302a, a white balance (WB) adjustment unit 302b, a gain adjustment unit 302c, a gamma correction unit 302d, a D/A converter 302e, a format change unit 302f, a sample memory 302g, and a still image memory 302h.

The synchronization unit 302a inputs the image signals received as the pixel information to three memories (not shown) provided per pixel, and sequentially updates and keeps values in the respective memories, associating with the pixel addresses of the light receiving unit 244f read by the reading unit 244g, and further synchronizes the image signals in the three memories as RGB image signals. The synchronization unit 302a sequentially outputs synchronized RGB image signals to the white balance adjustment unit 302b and also outputs some of RGB image signals to the sample memory 302g for image analysis such as brightness detection.

The white balance adjustment unit 302b automatically adjusts the white balance of the RGB image signal. More specifically, the white balance adjustment unit 302b automatically adjusts the white balance of the RGB image signal based on color temperature contained in the RGB image signal. Further, in the case where the sensor unit 244a adopts multi-line reading, gain variation between the multiple lines is adjusted.

The gain adjustment unit 302c adjusts the gain of the RGB image signal. The gain adjustment unit 302c outputs the RGB signal obtained after the gain adjustment to the gamma correction unit 302d, and also outputs some of the RGB signals to the still image memory 302h for displaying a still image, a magnified image or a highlight image.

The gamma correction unit 302d executes gradation correction (gamma correction) for the RGB image signal, corresponding to the display device 5.

The D/A converter 302e converts, to an analog signal, the RGB image signal obtained after the gradation correction which is output from the gamma correction unit 302d.

The format change unit 302f changes the image signal converted to the analog signal to a file format for a moving image such as high-vision system, and outputs the image to the display device 5.

The brightness detection unit 303 detects brightness level corresponding to each of the pixels based on the RGB image signal kept in the sample memory 302g, and records the detected brightness level in a memory provided inside, and further outputs the brightness level to the control unit 309. Further, the brightness detection unit 303 calculates a white balance adjustment value, a gain control value, and a light irradiation quantity based on the detected brightness level, and outputs the white balance adjustment value to the white balance adjustment unit 302b, the gain adjustment value to the gain adjustment unit 302c while outputting the light irradiation quantity to the light control unit 304.

The light control unit 304 sets a light type, a light quantity, light emission timing, etc. of the light generated by the light source device 4 based on the light irradiation quantity calculated by the brightness detection unit 303, and transmits a light source synchronizing signal including the set conditions to the light source device 4 under the control of the control unit 309.

The reading address setting unit 305 has a function to set pixels to be read and a reading order of the pixels on the light receiving surface of the sensor unit 244a by communicating with the control unit 271 inside the endoscope 2. The control unit 271 reads type information of the sensor unit 244a contained in the first EEPROM 274 and outputs the type information to the control device 3. In other words, the reading address setting unit 305 has a function to set the pixel address of the sensor unit 244a read by the AFE unit 244b. Further, the reading address setting unit 305 outputs the set address information of the reading target pixel to the synchronization unit 302a.

The drive signal generation unit 306 generates a drive timing signal (horizontal synchronizing signal (HD) and vertical synchronizing signals (VD)) for driving the endoscope 2, and transmits the signal to the timing generator 244d (image pickup device 244) via a prescribed signal line included in the FPGA 272 and the cable assemblies 224 and 245. The timing signal includes the address information of the reading target pixel, and may be superimposed on the setting data to be transmitted to the control unit 244e (timing generator 244d).

The input unit 307 receives inputs of various kinds of signals such as the operation instruction signals that instruct operations of the endoscope system 1, for
example, freeze, release, various kinds of image adjustments (highlight, electronic magnification, color tone, etc.) set by a front panel or a keyboard of the control device 3.

The storage unit 308 is implemented by a semiconductor memory such as a flash memory and a DRAM (Dynamic Random Access Memory). The storage unit 308 stores data including various kinds of programs for operating the endoscope system 1, various kinds of parameters necessary for operating the endoscope system 1, pattern information such as the test pattern signal to identify a location of abnormality (electrical signal corresponding to a specified display pattern), and the like. Also, the storage unit 308 stores the identification information and observation information of the control device 3. Here, the identification information includes individual information (ID) and a model year of the control device 3 as well as specification information and transmission rate information of the control unit 309.

The control unit 309 includes a CPU or the like, and executes drive control for the respective components including the endoscope 2 and the light source device 4, and also executes information input/output control for the respective components. The control unit 309 transmits, to the control unit 244e, the setting data for imaging control, the setting information for the test pattern signal at the time of determining abnormality, etc. via the FPGA 272 of the connector portion 27, and the signal and data required for the image pickup device 244 via a specified signal line included in the cable assemblies 224 and 245. The setting information for the test pattern includes information related to, for example, which test pattern signal is to be used in the case where there is a plurality of test patterns and at which component the test pattern signal is output for the image pickup device 244.

The reference clock generation unit 310 generates the reference clock signal which is to be the basis of operation in each of the components of the endoscope system 1, and supplies the generated reference clock signal to each of the components of the endoscope system 1. Note that either the clock generated by the reference clock generation unit 310 or the clock generated by the reference clock generation unit 273 may be used for the clock at the distal-end portion 24.

Next, a configuration of the light source device 4 will be described. The light source device 4 includes a light source 41, a light source driver 42, a rotary filter 43, a drive unit 44, a driving driver 45, and a light source controller 46.

The light source 41 includes a white LED (Light Emitting Diode), a xenon lamp or the like, and generates the white light under the control of the light source controller 46. The light source driver 42 causes the light source 41 to generate the white light by supplying current to the light source 41 under the control of the light source controller 46. The white light generated from the light source 41 is emitted from a distal end of the distal-end portion 24 via the rotary filter 43, a condenser lens (not shown), and the light guide 241.

The rotary filter 43 is disposed on an optical path of the white light generated by the light source 41, and rotated so as to pass only the light having a specified wavelength band of the white light generated by the light source 41. More specifically, the rotary filter 43 includes a red filter 431, a green filter 432, and a blue filter 433, which respectively pass the lights having the wavelength bands of red light (R), green light (G) and blue light (B). The rotary filter 43 is rotated, thereby sequentially passing the light having the wavelength bands of red, green and blue (for example, red: 600 nm to 700 nm, green: 500 nm to 600 nm, blue: 400 nm to 500 nm). This allows the white light generated from the light source 41 to sequentially emit any one of the red light, green light, and blue light having the narrowed wavelength band to the endoscope 2.

The drive unit 44 includes a stepping motor, a DC motor or the like, and rotates the rotary filter 43. The driving driver 45 supplies a specified current to the drive unit 44 under the control of the light source controller 46.

The light source controller 46 controls a current amount to be supplied to the light source 41 in accordance with a light source synchronizing signal transmitted from the light control unit 304. Also, the light source controller 46 rotates the rotary filter 43 by driving the drive unit 44 via the driving driver 45 under the control of the control unit 309.

The display device 5 has a function to receive, from the control device 3, the in-vivo image (an image for a moving image or an image for a still image) generated by the control device 3 via the video cable to display the in-vivo image. The display device 5 is formed of a liquid crystal, an organic EL (Electro Luminescence), or the like.

In the endoscope system 1 having the above-described configuration, abnormality location is identified in the case where abnormality occurs in a display image based an electrical signal in the electrical signal (image information) output from the endoscope 2. An exemplary way to identify the abnormality location may be a method in which the control unit 244e refers to the storage unit 244k and outputs a target test pattern signal based on the setting information of the test pattern signal from the control unit 309 and the test pattern signal is output via the timing generator 244d from any of the respective components (sensor unit 244a, P/S converter 244c, noise reduction unit 244h, AGC unit 244i, and A/D converter 244j). In this instance, the test pattern signal output from each of the components is transmitted to the operating unit 22 side via the signal line same as the signal line used to transmit the image of the endoscope 2. At this point, in the case where there is a plurality of the target components outputting the test pattern signal, each of the components individually outputs the test pattern signal.

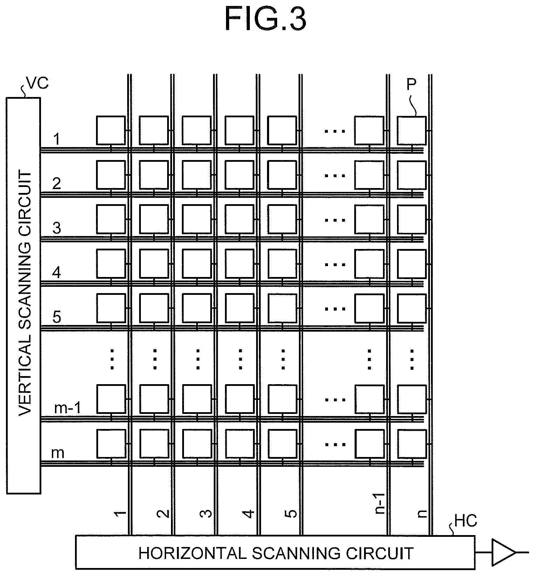

Now, input/output mode of the pixel signal (image signal) of the sensor unit 244a will be described. FIG. 3 is a circuit diagram illustrating a configuration of the sensor unit 244a of the endoscope system 1 according to the present embodiment. As described above, the sensor unit 244a includes: a light receiving unit 244f on which a plurality of pixels P is arranged in a two-dimensional matrix form and each of the plurality of pixels includes the photodiode that photoelectrically converts light from the optical system to output an electrical signal as image information and accumulates electric charge corresponding to the light quantity and an amplifier that amplifies the electric charge accumulated by the photodiode; and a reading unit 244g (vertical scanning circuit VC (row selection circuit) and horizontal scanning circuit HC (column selection circuit)) configured to read, as the image information, the electrical signal generated by the pixel P optionally set as the reading target from among the plurality of the pixels P of the light receiving unit. The vertical scanning circuit VC and the horizontal scanning circuit HC are respectively connected to each of the pixels P and configured to select the pixel. Further, the horizontal scanning circuit HC outputs the electrical signal from each of the pixels P to the outside.

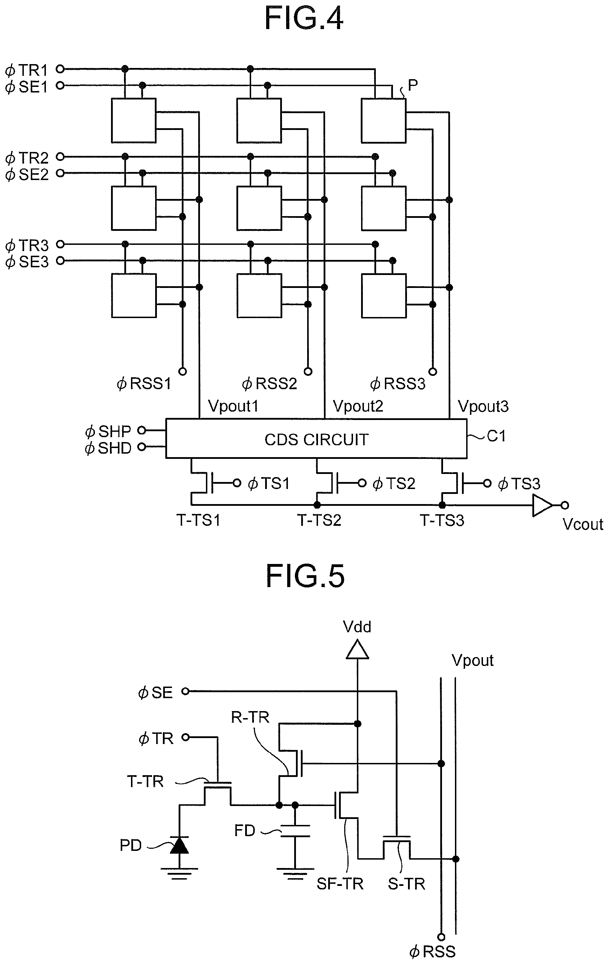

FIG. 4 is a circuit diagram schematically illustrating a configuration of the sensor unit 244a of the endoscope system 1 according to the present embodiment. FIG. 5 is a circuit diagram illustrating a configuration of a unit pixel of a light receiving unit 244f of the endoscope system 1 according the present embodiment. The pixel P includes: a photodiode PD that accumulates incident light after photoelectrically converting the incident light corresponding to the light quantity to a signal electric charge amount; a capacitor FD that converts the signal electric charge transmitted from the photodiode PD to a voltage level; a transfer transistor T-TR that transfers the signal electric charge accumulated in the photodiode PD to the capacitor FD during a period of ON state; a column control reset transistor R-TR that selects a column (N; N = 1, 2, 3, ···, n - 1, n) of the pixels P and releases the signal electric charge accumulated in the capacitor FD for resetting; a row selection transistor S-TR controlled to be turned ON in the case where a horizontal line including the unit pixel is selected as a reading target line (row, M; M = 1, 2, 3, ···, m - 1, m); and an output transistor SF-TR that outputs, to a specified signal line, the voltage level obtained from the signal electric charge and transmitted to the capacitor FD when the transfer transistor T-TR is in an ON state. The transfer transistor T-TR transfers the signal electric charge accumulated in the row selection transistor S-TR and the photodiode PD to the capacitor FD. Note that each of the pixels P is connected to the power source Vdd.

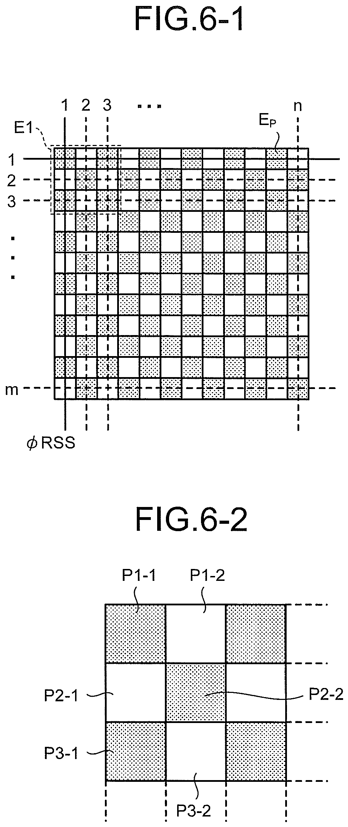

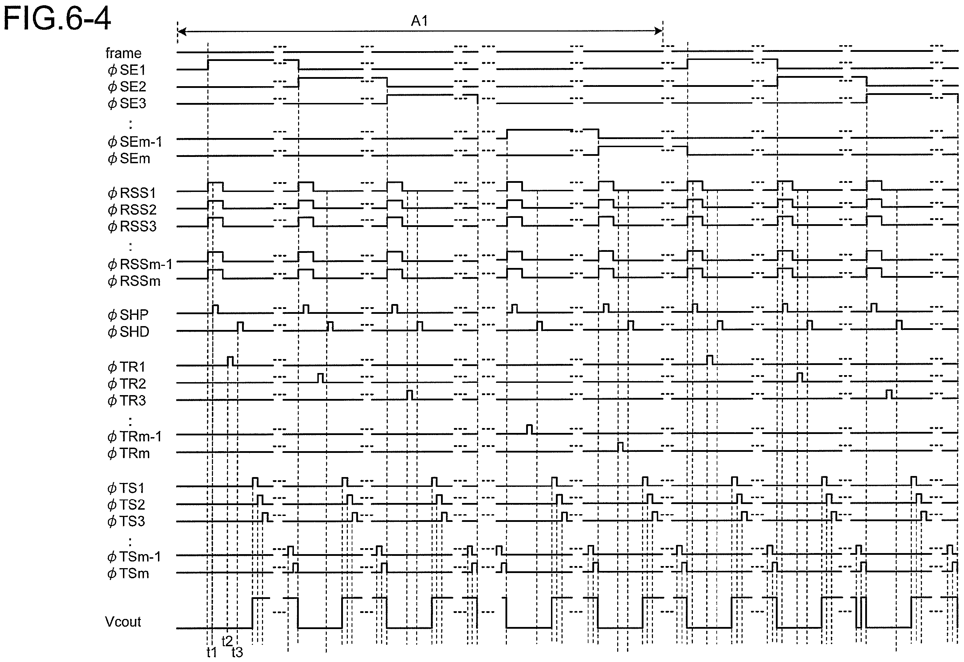

The operation of the sensor unit 244a in the pixel P having the above-described configuration will be described with reference to FIGS. 6-1 to 6-4. FIG. 6-1 is a diagram illustrating an image when a specified test pattern is output from a sensor unit 244a by pixel-by-pixel control. FIG. 6-2 is an enlarged diagram of an area E1 illustrated in FIG. 6-1. The test pattern according to the image illustrated in FIG. 6-1 is a pattern in that a signal level and a reset level are alternately output in one pixel unit for each of the pixels adjacent each other. In FIG. 6-2, the reference sign given to each pixel indicates a row and a column. For example, in the case of reference sign pixel P1-1, the number before a hyphen indicates the row (1; first line) and the number after the hyphen indicates the column (1; first column). In FIG. 6-2, the pixels (P1-1, P1-2, P2-1, P2-2, P3-1, P3-2) up to third row, second column are illustrated.

In the light receiving unit 244f, a row (M) is selected by a row selection pulse φSE from the vertical scanning circuit VC (row selection circuit), and the pixel signals of the pixels in the selected row are sequentially output as the pixel output voltage Vpout in accordance with the column number (N). For example, when the row M = 1 is selected, the pixel output voltage Vpout is output from each of the pixels in numerical order of the column numbers (N). After that, the pixel signal for the selected row (M) is output from each of the pixels. Thus, the image signal from each of the pixels P is output as the image signal from the sensor unit 244a to the outside after reducing the noise by using the correlated double sampling, for example. At this point, whether the pixel signal output from the pixel P includes the pixel information (signal electric charge of photodiode PD) is controlled by ON/OFF control of a column selection transistor R-TR.

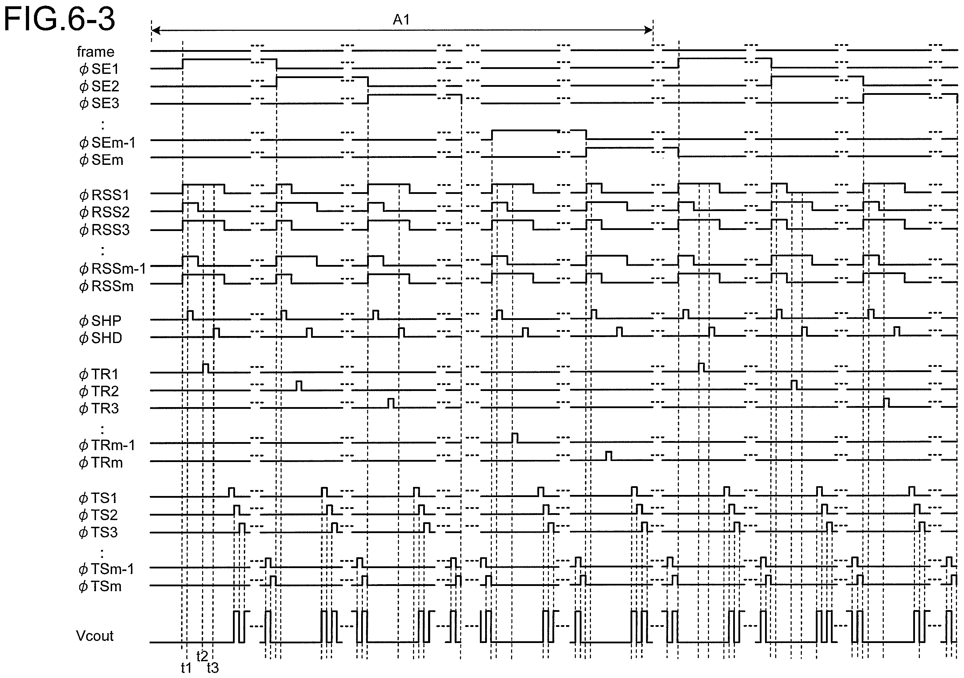

FIG. 6-3 is a timing chart illustrating an output mode when the test pattern corresponding to the image illustrating in FIG. 6-1 is output. Further, FIG. 6-4 is a timing chart illustrating an output mode when a captured image is output according to the related art. As illustrated in FIGS. 4, 5 and 6-3, the row selection pulse φSE is input to the row selection transistor S-TR and is kept at a high level while the target row is selected. Further, the reset pulse φRSS is input to the column control reset transistor R-TR, and whether to read the signal level or the reset level from the target pixel P is controlled by this inputting.

First, operation in the case where the pixel reads the signal level will be described with reference to FIG. 6-3. Note that the number given to each pulse φ indicates each row or column. Further, operation hereafter is performed under the control of the control unit 244e. After switching the row selection pulse φSE to the high level, the reset pulse φRSS is switched to the high level, and then the capacitor FD and the pixel output voltage Vpout are switched to the reset level. At this point, the electric charge transfer pulse φTR is controlled at a low level.

Here, the pixel output voltage Vpout is connected to a CDS circuit (correlated double sampling circuit) C1 (see FIG. 4) and is sampled by rise of the sample-and-hold pulse φSHP at time t1.

After completion of the reset level sampling by the sample-and-hold pulse φSHP, the reset pulse φRSS is switched to the low level. After the reset pulse φRSS is stabilized at the low level (time t2), the electric charge transfer pulse φTR is switched to the high level, and the voltage of the signal electric charge accumulated in the photodiode PD is converted at the capacitor FD, and also the pixel signal is output as the pixel output voltage Vpout at the output transistor SF-TR.

After the pixel signal is output to the pixel output voltage Vpout (time t3), a pixel signal level is sampled by a sample-and-hold pulse φSHD, and the image signal obtained by eliminating reset noise by a CDS circuit C1 is output as an output voltage Vcout to the outside of the sensor unit 244a by input of an output pulse φTS. The CDS circuit C1 is connected to a horizontal read line via the column control reset transistor R-TR. Further, the output voltage Vcout output during a period A1 constitutes one frame.

Next, operation in the case where the pixel reads the reset level will be described with reference to FIG. 6-3. After switching the row selection pulse φSE to the high level, the reset pulse φRSS is switched to the high level, and then the capacitor FD and the pixel output voltage Vpout are switched to the reset level. At this point, the electric charge transfer pulse φTR is controlled at the low level.

Here, the pixel output voltage Vpout is sampled by rise of a sample-and-hold pulse φSHP at time t1. After completion of reset level sampling by the sample-and-hold pulse φSHP, the reset pulse φRSS is controlled to be kept at the high level. In this state, the electric charge transfer pulse φTR is switched to the high level, and the signal electric charge accumulated in the photodiode PD is taken out. In this case, the capacitor FD is fixed at the reset level by the reset pulse φRSS, and therefore, the reset level is output to the pixel output voltage Vpout. After that, sampling of the pixel output voltage Vpout is executed by the sample-and-hold pulse φSHD (time t3).

The pixel signal and the reset level read from the CDS circuit C1 in each column are obtained as the pixel output voltage Vpout per row by sequentially switching ON/OFF the column control reset transistor R-TR for each of the columns. After completion of reading up to the column m, the row selection pulse φSE is switched to the low level to finish reading the row. Thus, the row selection pulse φSE is sequentially switched ON/OFF from the row selection pulses φSE1 to φSEm, thereby achieving to read one frame.

When outputting the test pattern illustrated in FIG. 6-1, the pixel signal level reading operation and the reset level reading operation are alternately performed for each row and column while operating the reset pulse φRSS. In the case of FIG. 6-1, the reset pulse φRSS is controlled such that odd number columns in a first row read the reset level, even number columns in the first row read the pixel signal level, the odd number columns in a second row read the pixel signal level, and the even number columns in the second row read the reset level.

By adopting the above-described configuration, the case of performing normal operation or the case of performing test pattern reading can be executed only by controlling operation of the reset pulse φRSS.

On the other hand, column-by-column control is not possible in the output mode when the captured image according to the related art is output as illustrated in FIG. 6-4, and therefore output operation is executed only by controlling the selected rows.

As described above, the column control reset transistor R-TR is capable of performing the column-by-column control, and the output mode of the signal from each of the pixels P arranged in the rows (M) and the columns (N) (electric charge amount transferred by the pixel) can be controlled by the pixel unit by executing the column-by-column control of the column control reset transistor R-TR for each of the pixels P. With this configuration, the columns in the selected horizontal line can be selected according to the present embodiment while only the horizontal line is selected for the reading target according to the related art. As a result, the degree of freedom in the output mode of the pixel P can be improved. Moreover, according to the present embodiment, normal reading control (outputting signals including pixel information from all of pixels), test pattern switching control, and pattern control (display mode) for the test pattern can be executed only by controlling the operation of the reset pulse φRSS.

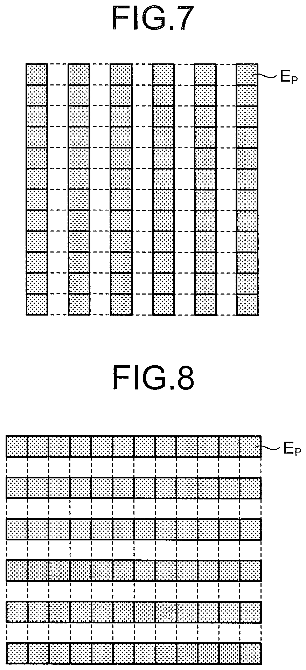

FIGS. 7 and 8 are schematic diagrams each illustrating exemplary images corresponding to the test pattern signals in the endoscope system 1 according to the present embodiment. An image to be displayed can be set in the test pattern signal by the above-described pixel-by-pixel control. For instance, a latticed pattern may be formed by alternately setting ON and OFF of inclusion of the pixel information as illustrated in FIG. 6-1. In this instance, the shaded portions in FIG. 6-1 correspond to the pixel signals not including the pixel information. Also here, the latticed pattern can be formed in an area Ep corresponding to one pixel in the image to be displayed by setting inclusion and non-inclusion of the pixel information by the pixel unit.

Inclusion and non-inclusion of the pixel information may be alternately set in the column direction as illustrated in FIG. 7, or inclusion and non-inclusion of the pixel information may be alternately set in the row direction as illustrated in FIG. 8. In this instance, inclusion and non-inclusion of the pixel information may be set in the area Ep corresponding to one pixel or may be set by the unit of plural pixels. Also, there may be other options in which setting is made such that color tone changes stepwise or each pixel is colored different.

Thus, by controlling the output mode of the signal of the respective pixels P arranged in the rows (M) and columns (N), it is possible to identify abnormality at the sensor unit 244a by the test pattern signal at the time of determining abnormality of the sensor unit 244a, and also abnormality determination can be executed by the pixel unit. Further, the test pattern may be used for adjusting the sensor unit 244a as well.

### (Sampling Pulse Phase Adjustment at A/D converter)

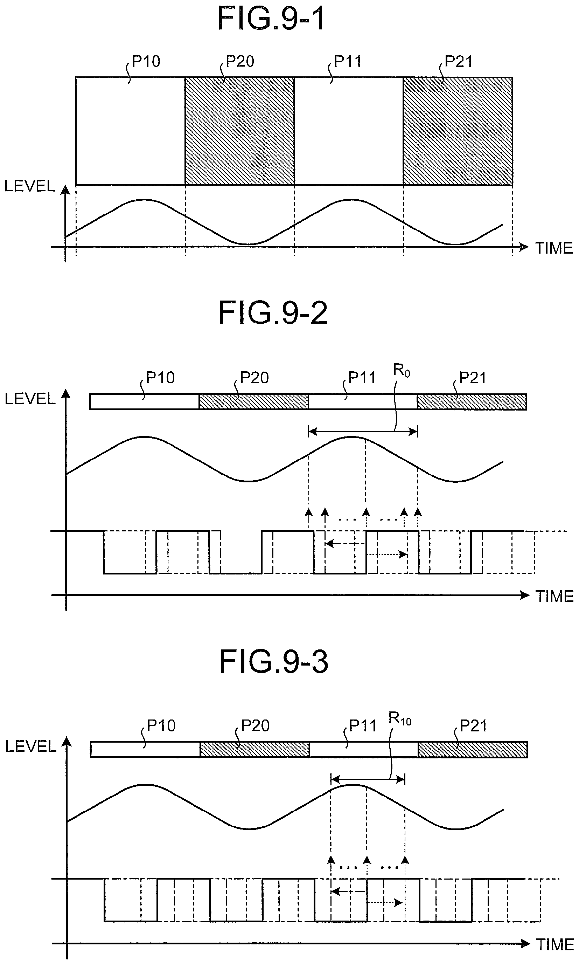

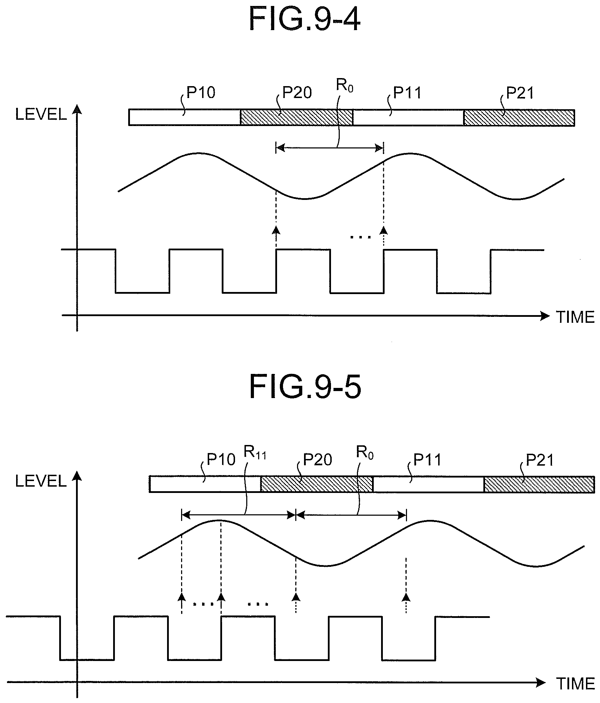

In the case of outputting the above-described test pattern signal, phase adjustment for the pulse at the A/D converter 244j can be executed, for example, by using the test patterns having different brightness level between adjacent pixels. FIGS. 9-1 to 9-5 are explanatory diagrams illustrating exemplary use modes of the test pattern signal according to the present embodiment, where pixel patterns and pulses that determine analog video signal waveform and/or sampling timing (hereinafter referred to as sampling pulse") are illustrated. The description for FIGS. 9-1 to 9-5 will be given, provided that the test pattern is output from the distal-end portion 24 at the time of adjusting the sampling pulse to an optimal phase when analog-digital conversion is performed by the A/D converter 244j in the configuration where an analog video signal is output from the endoscope 2. However, this may be applied to any location that performs analog-digital conversion at the distal-end portion 24, operating unit 22, connector portion 27, and control device 3.

Adjusting the sampling pulse phase at the A/D converter 244j is to adjust the sampling pulse to the optimal position (phase) inside the video signal of one pixel by outputting a test pattern in which the brightness level is highlighted in every other pixel. For instance, the optimal position of the sampling pulse is a position at a peak point of the analog video signal waveform, obtained by cutting frequency components higher than a maximum video signal frequency by using a lowpass filter in the arrangement of adjacent pixels P10, P11, P20 and P21 having different brightness levels, as illustrated in FIG. 9-1.

More specifically, the phase of the sampling pulse is adjusted to the highest signal level point (peak point) in the analog video signal of one pixel (FIG. 9-2). A method of this adjustment is to sequentially change the phase of the sampling pulse within a one-pixel video signal transfer period R₀ by short step (indicated by dotted arrows or alternate long and short dash line arrows in the drawing) and grasp the level of the video signal obtained by analog-digital conversion in each step. The level of the video signal is detected at the FPGA 272 and transmitted to the control device 3. After completion of transmission, an instruction for changing the phase is transmitted from the control device 3 to the FPGA 272. The above operation is repeated within a range of one pixel. A step change instruction for the phase change is executed by the communication from the control device 3 to the FPGA 272 of the endoscope 2.

The step having the highest video signal level is determined as the optimal sampling pulse position (phase) based on the detection results of the video signal level, and the determined optimal position is stored in the second EEPROM 275 or the storage unit 308 as an adjustment value, so that the phase position of the sampling pulse is read and set at the time starting the system. Note that this sampling pulse adjustment is performed asynchronously with the video signal.

Here, in the case where the sampling pulse is swept in the phase step sufficiently shorter compared to the one-pixel video signal transfer period R₀ within the one-pixel video signal transfer period R₀ to acquire the level of the video signal, it takes some time to scan all the steps within the one-pixel video signal transfer period R₀ by the number of steps in order to detect the optimal phase from the acquired video levels. In other words, it takes a long time in the case where the number of steps is increased by making each scanning step short for sake of improving sampling accuracy or in the case where the system has a long one-pixel video signal transfer period R₀.

In view of the situation, the above-described adjustment method may be suitably modified as described below. An adjustment method is to create groups for respective video signal input timings to the A/D converter 244j, and limit a scanning range of the sampling pulse to a video signal transfer period R₁₀ shorter compared to the video signal transfer period R₀ for each of the groups, thereby reducing the adjustment time (FIG. 9-3). Groups may be created, for example, per model of the endoscope 2. Note that it is preferable that groups be created per model of the endoscope 2 because a delay amount of the video signal varies depending on a cable length, a type of image sensor (image pickup device 244), and so on. Further, the scanning range per group is stored in the second EEPROM 275 inside the endoscope 2 or the storage unit 308 as an adjustment parameter, and the scanning range of the sampling pulse is read at the time of executing adjustment to control adjustment operation, and is set in software of the control device 3.

By adopting the above-described method, the scanning range in the case of adjusting the phase of the sampling pulse per group can be minimized within the one-pixel video signal transfer period R₁₀. As a result, the adjustment time can be considerably shortened.

Also, in the endoscope 2, a cable transmission distance for a signal varies because the length of the inserting portion 21 varies depending on a used region of a human body. For instance, in the endoscope including the A/D converter mounted on the connector portion 27, the video signal input timing to the A/D converter varies due to the above-described reason, but in the case of executing the same adjusting method for the inserting portion having the longest length and the inserting portion having the shortest length, the video signal transfer period R₀ is deviated and an obtained position of the sampling pulse may differs from the optimal position (FIG. 9-4).

In order to avoid obtaining such a position of the sampling pulse different from the optimal position, in the type of the endoscope 2 where no optimal position exists within the scanning range in accordance with the cable transmission distance, adjustment is executed by setting the scanning range of the sampling pulse to a video signal transfer period R11 illustrated in FIG. 9-4 (FIG. 9-5) and earlier than the video signal transfer period R₀ by one pixel portion. The data indicating whether to set the scanning range one pixel portion earlier is stored in the second EEPROM 275 inside the endoscope 2 or the storage unit 308 as the adjustment parameter, and the scanning range of the sampling pulse is read at the time of executing adjustment to control the adjustment operation, and is set in the software of the control device 3.

Note that the above-described method is not limited to the sampling pulse phase adjustment at the A/D converter 244j. For instance, the method may be applied to detect an optimal phase of a sampling pulse at the noise reduction unit 244h and the AGC unit 244i inside the AFE unit 244b.

### (Correction Data Format of Digital Video Data)

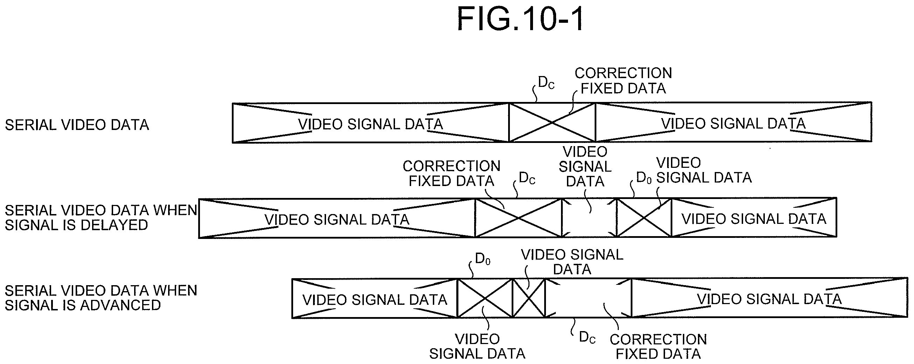

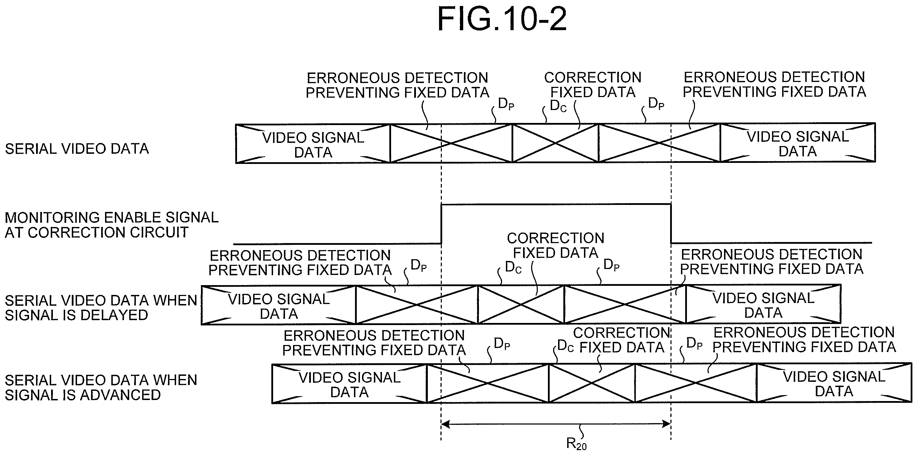

FIGS. 10-1 and 10-2 are explanatory diagrams illustrating exemplary use modes of signal transmission, where timing charts of the respective signals (data) are illustrated. In the signal transmission according to the present embodiment, video signals may be serialized and transferred to reduce the transmission line in the case where a circuit for converting an analog video signal to a digital video signal (A/D converter 244j) is provided distant from a circuit for executing image processing (image processing unit 302). Also, in the case where digital video signals are output from the A/D converter 244j in parallel, the signals may be serialized once by the FPGA 272, and in the case where the transmission distance is long, the signals may be transmitted by an LVDS (Low voltage differential signaling) system whereby amplitude can be suppressed.

Here, according to the above-described video signal transmission, phase shift may occur at the image position and in the signal at the image pickup device 244 due to image position displacement or signal delay at the image processing unit 302 on the receiving side. A method of correcting such a phase shift is to superimpose correction fixed data Dc, which is to be positional information for images and phase adjustment information, inside the serialize video data. Correction is executed by detecting the correction fixed data and detecting displacement of the image position and phase shift.

However, in the case where the video data has the same data pattern as the correction fixed data at the time of detecting the correction fixed data (e.g., video signal data D₀), a correction circuit inside the image processing unit 302 may erroneously recognize the video signal data D₀ as the correction fixed data Dc and erroneous correction may be made (FIG. 10-1). In FIG. 10-1, respective serialized serial video data are illustrated in the respective cases where a signal is delayed and a signal is advanced.

There is a method to avoid such an erroneous correction caused by erroneous recognition, in which only the vicinity of the timing when the correction fixed data Dc (correction fixed data monitoring period R₂₀) is transferred is monitored by the correction circuit (e.g., image processing unit 302 or control unit 309) on the receiving side (see FIG. 10-2). In other timings, mask control is performed such that the correction fixed data Dc is not detected at the correction circuit. Further, erroneous detection preventing fixed data Dp having a data pattern different from the correction fixed data Dc is superimposed on the periphery of the correction fixed data Dc so as to avoid erroneous detection by the correction circuit. This enables the correction circuit to avoid erroneously detecting the video data as the correction fixed data Dc and surely detect and correct the displacement of image position and phase shift. Note that the data pattern of the erroneous detection preventing fixed data Dp may be the data pattern such as "A55A" in hexadecimal notation or a simple clock signal.

The above-described method of avoiding correction by the erroneous recognition is not limited between the A/D converter 244j outputting the serial video data and the image processing unit 302. For example, the method may be used for delay correction of the transmission distance and enhancement of resistance against disturbance noise, etc. in control signal communication between two circuits.

According to the above-described present embodiment, the control unit 244e performs the pixel-by-pixel control for the output mode of the electrical signal output from the respective pixels P so as to output the electrical signal (test pattern signal) corresponding to the prescribed display pattern, thereby achieving to identify a location of abnormality inside the endoscope 2, particularly details of the abnormality location at the sensor unit 244a. Further, the control unit 244e is configured to output the test pattern signal to any of the following components (sensor unit 244a, P/S converter 244c, noise reduction unit 244h, AGC unit 244i, and A/D converter 244j) via the timing generator 244d. As a result, optical and electrical evaluation is performed based on the obtained signal and it is possible to identify the location of abnormality at the image pickup device 244 in detail.

In this case, the optical and electrical evaluation based on the obtained signal may be conducted by an observer by using images and the like displayed on the display device, or may be automatically performed on the control device 3 side by comparing the test pattern signal obtained from the endoscope 2 side with the test pattern signal stored in the storage unit 308.

Also, according to the present embodiment, the test pattern signals are output from the respective components in parallel and can be simultaneously output splitting on the screen. Accordingly, it is possible to identify abnormal locations in the plurality of components at the same time.

Also, according to the above-described embodiment, electrical signal output may be controlled by performing ON/OFF control of the buffer output at the P/S converter 244c or the operating unit 22. With this configuration, the electrical signals respectively output from the operating unit 22 and the distal-end portion 24 can be separated. Particularly, this configuration can be used to examine EMC (Electro-Magnetic Compatibility; electromagnetic compatibility) of the operating unit 22 and the distal-end portion 24.

According to the above-described present embodiment, it has been described that the light source device 4 adopts the frame sequential method, including the rotary filter 43, but the light source device may also adopt the simultaneous method without the rotary filter 43 as long as a color filter is included on the image pickup device 244 side.

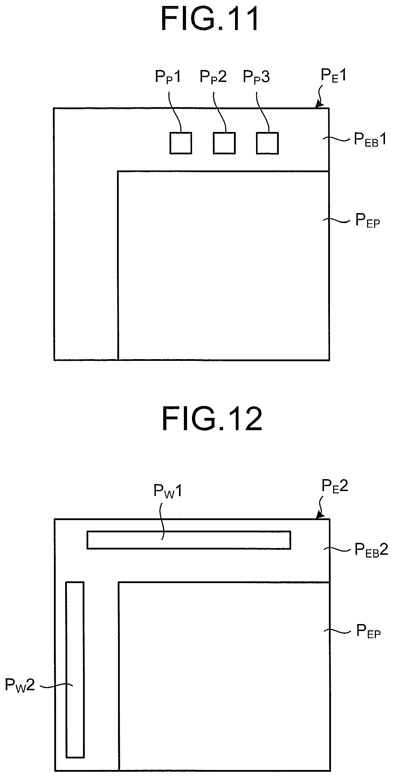

FIG. 11 is a schematic diagram illustrating a light receiving unit according to a modified example 1 of the present embodiment. According to the modified example 1, a pixel array area P_{E}1 of the light receiving unit includes: an effective pixel area P_{EP} where the pixels used for actual imaging are arrayed; and an optical black area P_{EB}1 provided around the effective pixel area P_{EP} where the pixels used for noise correction are arrayed and the pixels are shielded.

According to the modified example 1, three test pixels P_{P}1, P_{P}2 and P_{P}3 capable of receiving the light (not shielded) are provided on the optical black area P_{EB}1. The three test pixels P_{P}1, P_{P}2 and P_{P}3 are arranged at specified intervals, for example, at the interval corresponding to one pixel. The sensor unit 244a reads the optical black area P_{EB}1 by the normal reading method.

By arranging the three test pixels P_{P}1, P_{P}2 and P_{P}3 at the specified intervals, crosstalk level can be checked. Also, since the interval between the pixels is clear, optical resolution can be checked without using the optical system. A center position of the effective pixel area P_{EP} can be detected by placing one of the three test pixels at the center position of the effective pixel area (center portion of one side of the rectangular-shaped effective pixel area). With this configuration, it is possible to identify the abnormality location far more in detail.

FIG. 12 is a schematic diagram illustrating the light receiving unit according to a modified example 2 of the present embodiment. According to the modified example 2, a pixel array area P_{E}2 of the light receiving unit includes the above-described effective pixel area P_{EP}, and an optical black area P_{EB}2 provided around the effective pixel area P_{EP} where the pixels used for noise correction are arrayed and the pixels are shielded.

According to the modified example 2, the optical black area P_{EB}2 includes two test pixel areas P_{W}1 and P_{W}2 capable of receiving the light (not shielded). The two test pixel areas P_{W}1 and P_{W}2 have an approximate rectangular-shape and extend in directions orthogonal to each other.

Optical distortion (distortion) can be checked by using image information obtained from the test pixel areas P_{W}1 and P_{W}2 that have an approximate rectangular-shape and extend. Also, since the two test pixel areas P_{W}1 and P_{W}2 are orthogonally arranged, distortion of the two orthogonal directions can be detected in the effective pixel area P_{EP}. With this configuration, it is possible to identify the abnormality location far more in detail.

The test pixels P_{P}1, P_{P}2, P_{P}3 and the test pixel areas P_{W}1, P_{W}2 according to the above-described modified examples 1 and 2 can be optionally combined. Also, the arranged position of each of the test pixels can be suitably adjusted.

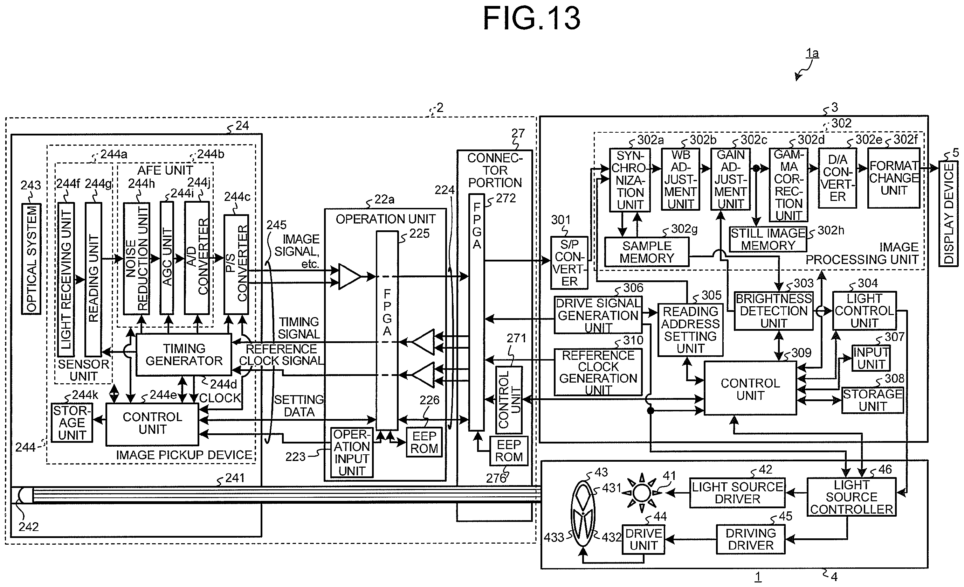

FIG. 13 is a block diagram illustrating a functional configuration in the main part of an endoscope system according to a modified example 3 of the present embodiment. According to the above-described embodiment, it has been described that the test pattern signal is output inside the distal-end portion 24, but the test pattern signal may be output from the operating unit like the modified example 3. The operating unit 22a according to the modified example 3 includes the above-described operation input unit (switch) 223, an FPGA 225, and an EEPROM 226 that records configuration data of the FPGA 225. Further, the connector portion 27 is provided with an EEPROM 276 storing the endoscope individual data including the configuration data and imaging information of the FPGA 272. The FPGA 225 outputs the test pattern signal under the control of the control unit 309. The control device 3 allows the display device 5 to display an image based on the test pattern signal output from the FPGA 225. The operating unit 22a is electrically connected to each of the distal-end portion 24 (image pickup device 244) and the control device 3, and functions as the relay processing unit that relays the electrical signal. Note that the operating unit 22a and the control device 3 are electrically connected via the connector portion 27. Further, the test pattern signal may be output from the FPGA 272 of the connector portion 27. Also, the FPGA 272 may be incorporated to the FPGA 225.

According to the above-described modified example 3, abnormality at the operating unit may be identified, in addition to the above-described embodiments. With this configuration, it is possible to identify the abnormality location far more in detail. Abnormality at a component other than the operating unit can be also identified by outputting the test pattern signal from the component in the case the component has the configuration capable of outputting the test pattern signal (for example, connector portion 27).

### Industrial Applicability

As described above, the imaging apparatus and the imaging system according to the present invention are useful to identify the abnormality location inside the imaging apparatus in detail.

### Reference Signs List

- 1, 1a: endoscope system
- 2: endoscope
- 3: control device
- 4: light source device
- 5: display device
- 21: inserting portion
- 22, 22a: operating unit
- 23: universal cord
- 24: universal cord
- 25: bending portion
- 26: flexible tube
- 27: connector portion
- 41: light source
- 42: light source driver
- 43: rotary filter
- 44: drive unit
- 45: driving driver
- 46: light source controller
- 221: bending knob
- 222: treatment instrument inserting portion
- 223: switch
- 224, 245: cable assembly
- 225, 272: FPGA
- 226, 276: EEPROM
- 241: light guide
- 242: illumination lens
- 243: optical system
- 244: image pickup device
- 244a: sensor unit
- 244b: analog front end
- 244c: P/S converter
- 244d: timing generator
- 244e, 271, 309: control unit
- 244f: light receiving unit
- 244g: reading unit
- 244h: noise reduction unit
- 244i: AGC unit
- 244j: A/D converter
- 244k, 308: storage unit
- 273, 310: reference clock generation unit
- 274: first EEPROM
- 275: second EEPROM
- 302: image processing unit
- 303: brightness detection unit
- 304: light control unit
- 305: reading address setting unit
- 306: drive signal generation unit
- 307: input unit

## Claims

1. An imaging apparatus (244) comprising:
a sensor unit (244a) having a light receiving unit (244f) provided with a plurality of pixels each adapted to photoelectrically convert received light to a pixel voltage signal level to generate an electrical signal after photoelectric conversion, and capable of reading the electrical signal generated by the light receiving unit as image information;
a control unit (244e) configured to control an output mode of the electrical signal output by each pixel, on a pixel-by-pixel basis, output the electrical signal corresponding to a specified display pattern, and set the pixels to a pixel voltage reset level after photoelectric conversion;
a signal processing unit (244b) configured to perform signal processing on the electrical signal output from the sensor unit (244a); and
a transmission unit (244c) configured to transmit a processed signal processed by the signal processing unit (244b) to outside,
**characterized in that**
the specified display pattern is a test pattern, and,
when the electrical signal corresponding to the test pattern is to be output, the control unit (244e) is configured to perform pixel signal level reading operation and reset level reading operation alternately for each row and column of the plurality of pixels provided in the light receiving unit.

2. The imaging apparatus (244) according to claim 1, wherein the signal processing unit (244b) includes:
a noise reduction unit (244h) configured to reduce a noise component contained in the electrical signal;
an adjustment unit (302c) configured to adjust a gain of the electrical signal to keep a constant output level; and
an A/D converter (244j) configured to perform analog-digital conversion on the electrical signal output via the adjustment unit,
wherein the control unit (244e) is configured to select one or a plurality of units from among the noise reduction unit (244h), the adjustment unit (302c), the A/D converter (244j) and the transmission unit (244c), and cause each of the selected units to output the electrical signal corresponding to the test pattern.

3. The imaging apparatus (244) according to claim 2, further comprising a lowpass filter configured to input the electrical signal, cut a frequency component higher than a specified frequency in the electrical signal, and output the electrical signal to the A/D converter (244j),
wherein the control unit (244e) is configured to set a phase of a sampling pulse for performing the analog-digital conversion by the A/D converter (244j) to a phase exhibiting a peak value of the electrical signal that has passed the lowpass filter and corresponds to the test pattern.

4. The imaging apparatus (244) according to claim 3, further comprising a storage unit (308) configured to store the phase of the sampling pulse having been set.

5. The imaging apparatus (244) according to claim 1, wherein
the sensor unit (244a) includes the light receiving unit (244f) having an effective pixel area (P_{EP}) and an optical black area (P_{EB}1,P_{EB}2) provided at a periphery of the effective pixel area, and
a part of the optical black area includes a pixel (P_{W}1, P_{W}2) capable of receiving light.

6. The imaging apparatus (244) according to claim 5, wherein three pixels capable of receiving the light in the optical black area are arranged at specified intervals.

7. The imaging apparatus (244) according to claim 5, wherein one of the three pixels capable of receiving the light in the optical black area is arranged at a center portion of at least one side of a rectangular shaped effective pixel area.

8. The imaging apparatus (244) according to claim 5, wherein pixels capable of receiving the light in the optical black area constitute two pixel areas which have an approximate rectangular-shape and extend in directions orthogonal to each other.

9. An imaging system comprising:
the imaging apparatus (244) of any preceding claim; and
a processing device (3) electrically connected to the imaging apparatus (244) and configured to generate image data based on the processed signal transmitted from the transmission unit (244c).

10. The imaging system according to claim 9, further comprising a relay processing unit electrically connected to each of the imaging apparatus and the processing device and configured to relay the processed signal,
wherein the processing device is configured to cause the relay processing unit to output the processed signal corresponding to the test pattern.

## Patentansprüche

1. Abbildungsvorrichtung (244), umfassend:
eine Sensoreinheit (244a), die eine Lichtempfangseinheit (244f) aufweist, die mit einer Mehrzahl Pixel versehen ist, von denen jedes dazu angepasst ist, empfangenes Licht fotoelektrisch in einen Pixelspannungssignalpegel umzuwandeln, um nach der fotoelektrischen Umwandlung ein elektrisches Signal zu erzeugen, und fähig ist, das von der Lichtempfangseinheit erzeugte elektrische Signal als Bildinformation zu lesen;
eine Steuerungseinheit (244e), die dazu ausgelegt ist, einen Ausgangsmodus des Ausgangs des elektrischen Signals durch jedes Pixel auf einer Pixel-für-Pixel-Basis zu steuern, das elektrische Signal entsprechend einem spezifizierten Anzeigemuster auszugeben und die Pixel nach der fotoelektrischen Umwandlung auf einen Pixelspannungszurücksetzpegel einzustellen;
eine Signalverarbeitungseinheit (244b), die dazu ausgelegt ist, die Signalverarbeitung auf dem Ausgang des elektrischen Signals aus der Sensoreinheit (244a) durchzuführen; und
eine Sendeeinheit (244c), die dazu ausgelegt ist, ein von der Signalverarbeitungseinheit (244b) verarbeitetes Signal nach außen zu senden,
**dadurch gekennzeichnet, dass**
das spezifizierte Anzeigemuster ein Testmuster ist, und,
wenn das elektrische Signal entsprechend dem Testmuster auszugeben ist, die Steuerungseinheit (244e) dazu ausgelegt ist, abwechselnd eine Pixelsignalpegelleseoperation und eine Zurücksetzpegelleseoperation für jede Reihe und Spalte der in der Lichtempfangseinheit vorgesehenen Mehrzahl Pixel durchzuführen.

2. Abbildungsvorrichtung (244) nach Anspruch 1, wobei die Signalverarbeitungseinheit (244b) umfasst:
eine Rauschverringerungseinheit (244h), die dazu ausgelegt ist, eine in dem elektrischen Signal enthaltene Rauschkomponente zu verringern;
eine Einstelleinheit (302c), die dazu ausgelegt ist, eine Verstärkung des elektrischen Signals einzustellen, um einen konstanten Ausgangspegel zu halten; und
einen A/D-Wandler (244j), der dazu ausgelegt ist, über die Einstelleinheit eine Analog-Digital-Umwandlung auf dem Ausgang des elektrischen Signals durchzuführen,
wobei die Steuerungseinheit (244e) dazu ausgelegt ist, eine oder eine Mehrzahl von Einheiten unter der Rauschverringerungseinheit (244h), der Einstelleinheit (302c), dem A/D-Wandler (244j) und der Sendeeinheit (244c) auszuwählen und zu bewirken, dass jede der ausgewählten Einheiten das elektrische Signal entsprechend dem Testmuster ausgibt.

3. Abbildungsvorrichtung (244) nach Anspruch 2, ferner umfassend einen Tiefpassfilter, der dazu ausgelegt ist, das elektrische Signal einzugeben, eine Frequenzkomponente zu schneiden, die höher ist als eine spezifizierte Frequenz in dem elektrischen Signal, und das elektrische Signal an den A/D-Wandler (244j) auszugeben,
wobei die Steuerungseinheit (244e) dazu ausgelegt ist, eine Phase eines Abtastimpulses zum Durchführen der Analog-Digital-Umwandlung durch den A/D-Wandler (244j) auf eine Phase einzustellen, die einen Spitzenwert des elektrischen Signals zeigt, das den Tiefpassfilter passiert hat und dem Testmuster entspricht.

4. Abbildungsvorrichtung (244) nach Anspruch 3, ferner umfassend eine Speichereinheit (308), die dazu ausgelegt ist, die Phase des Abtastimpulses, die eingestellt wurde, zu speichern.

5. Abbildungsvorrichtung (244) nach Anspruch 1, wobei
die Sensoreinheit (244a) die Lichtempfangseinheit (244f) umfasst, die eine effektive Pixelfläche (P_{EP}) und eine optische Schwarzfläche (P_{EB}1, P_{EB}2) aufweist, die an einer Peripherie der effektiven Pixelfläche vorgesehen sind, und
einen Teil der optischen Schwarzfläche ein Pixel (P_{W}1, P_{W}2) umfasst, das fähig ist, Licht zu empfangen.

6. Abbildungsvorrichtung (244) nach Anspruch 5, wobei drei Pixel, die fähig sind, Licht in dem optischen Schwarzbereich zu empfangen, in spezifizierten Intervallen angeordnet sind.

7. Abbildungsvorrichtung (244) nach Anspruch 5, wobei eins der drei Pixel, die fähig sind, Licht in dem optischen Schwarzbereich zu empfangen, in einem mittleren Abschnitt von mindestens einer Seite einer rechtwinklig geformten effektiven Pixelfläche angeordnet sind.

8. Abbildungsvorrichtung (244) nach Anspruch 5, wobei die Pixel, die fähig sind das Licht in der optischen Schwarzfläche zu empfangen, zwei Pixelflächen bilden, die eine ungefähre rechtwinklige Form aufweisen und sich in Richtungen orthogonal zueinander erstrecken.

9. Abbildungssystem, umfassend:
die Abbildungsvorrichtung (244) nach einem vorhergehenden Anspruch; und
eine Verarbeitungseinrichtung (3), die elektrisch mit der Bildvorrichtung (244) verbunden und dazu ausgelegt ist, Bilddaten basierend auf dem verarbeiteten Signal, das von der Sendeeinheit (244c) gesendet wurde, zu erzeugen.

10. Abbildungssystem nach Anspruch 9, das ferner eine Relaisverarbeitungseinheit umfasst, die elektrisch mit jeder der Abbildungsvorrichtung und der Verarbeitungseinrichtung verbunden und dazu ausgelegt ist, das verarbeitete Signal weiterzugeben,
wobei die Verarbeitungseinheit dazu ausgelegt ist, zu bewirken, dass die Relaisverarbeitungseinheit das dem Testmuster entsprechende verarbeitete Signal ausgibt.

## Revendications

1. Appareil d'imagerie (244) comprenant :
une unité de capteur (244a) ayant une unité de réception de lumière (244f) ayant une pluralité de pixels, chacun adapté pour convertir photo-électriquement la lumière reçue en un niveau de signal de tension de pixel pour générer un signal électrique après la conversion photoélectrique, et capable de lire le signal électrique généré par l'unité de réception de lumière en tant qu'informations d'image ;
une unité de commande (244e) configurée pour commander un mode de sortie du signal électrique délivré par chaque pixel, sur une base pixel par pixel, délivrer le signal électrique correspondant à un motif d'affichage spécifié, et régler les pixels à un niveau de réinitialisation de tension de pixel après la conversion photoélectrique ;
une unité de traitement de signal (244b) configurée pour réaliser un traitement de signal sur le signal électrique délivré par l'unité de capteur (244a) ; et
une unité d'émission (244c) configurée pour émettre un signal traité, qui est traité par l'unité de traitement de signal (244b), vers l'extérieur,
**caractérisé par le fait que**
le motif d'affichage spécifié est un motif d'essai, et
lorsque le signal électrique correspondant au motif d'essai doit être délivré, l'unité de commande (244e) est configurée pour réaliser une opération de lecture de niveau de signal de pixel et une opération de lecture de niveau de réinitialisation de manière alternée pour chaque rangée et colonne de la pluralité de pixels situés dans l'unité de réception de lumière.

2. Appareil d'imagerie (244) selon la revendication 1, dans lequel l'unité de traitement de signal (244b) comprend :
une unité de réduction de bruit (244h) configurée pour réduire une composante de bruit contenue dans le signal électrique ;
une unité d'ajustement (302c) configurée pour ajuster un gain du signal électrique pour maintenir un niveau de sortie constant ; et
un convertisseur analogique/numérique, A/N, (244j) configuré pour réaliser une conversion analogique/numérique sur le signal électrique délivré par l'intermédiaire de l'unité d'ajustement,
l'unité de commande (244e) étant configurée pour sélectionner une ou plusieurs unités parmi l'unité de réduction de bruit (244h), l'unité d'ajustement (302c), le convertisseur A/N (244j) et l'unité d'émission (244c), et amener chacune des unités sélectionnées à délivrer le signal électrique correspondant au motif d'essai.

3. Appareil d'imagerie (244) selon la revendication 2, comprenant en outre un filtre passe-bas configuré pour entrer le signal électrique, couper une composante de fréquence supérieure à une fréquence spécifiée dans le signal électrique, et délivrer le signal électrique au convertisseur A/N (244j),
l'unité de commande (244e) étant configurée pour régler une phase d'une impulsion d'échantillonnage pour réaliser la conversion analogique/numérique par le convertisseur A/N (244j) à une phase présentant une valeur de crête du signal électrique qui est passé par le filtre passe-bas et correspond au motif d'essai.

4. Appareil d'imagerie (244) selon la revendication 3, comprenant en outre une unité de stockage (308) configurée pour stocker la phase de l'impulsion d'échantillonnage ayant été réglée.

5. Appareil d'imagerie (244) selon la revendication 1, dans lequel
l'unité de capteur (244a) comprend l'unité de réception de lumière (244f) ayant une zone de pixel effective (P_{EP}) et une zone noire optique (P_{EB}1, P_{EB}2) située à une périphérie de la zone de pixel effective, et
une partie de la zone noire optique comprend un pixel (P_{W}1, P_{W}2) capable de recevoir de la lumière.

6. Appareil d'imagerie (244) selon la revendication 5, dans lequel trois pixels capables de recevoir la lumière dans la zone noire optique sont disposés à des intervalles spécifiés.

7. Appareil d'imagerie (244) selon la revendication 5, dans lequel l'un des trois pixels capables de recevoir la lumière dans la zone noire optique est disposé à une partie centrale d'au moins un côté d'une zone de pixel effective de forme rectangulaire.

8. Appareil d'imagerie (244) selon la revendication 5, dans lequel des pixels capables de recevoir la lumière dans la zone noire optique constituent deux zones de pixel qui ont une forme approximativement rectangulaire et s'étendent dans des directions orthogonales l'une à l'autre.

9. Système d'imagerie comprenant :
l'appareil d'imagerie (244) selon l'une quelconque des revendications précédentes ; et
un dispositif de traitement (3) relié électriquement au dispositif d'imagerie (244) et configuré pour générer des données d'image sur la base du signal traité émis par l'unité d'émission (244c).

10. Système d'imagerie selon la revendication 9, comprenant en outre une unité de traitement de relais reliée électriquement à chacun parmi l'appareil d'imagerie et le dispositif de traitement et configurée pour relayer le signal traité,
le dispositif de traitement étant configuré pour amener l'unité de traitement de relais à délivrer le signal traité correspondant au motif d'essai.
